(19) Europäisches Patentamt
European Patent Office
Office européen des brevets

(11) EP 4 603 476 A1

(12) **EUROPEAN PATENT APPLICATION**
published in accordance with Art. 153(4) EPC

(43) Date of publication:
**20.08.2025 Bulletin 2025/34**

(21) Application number: **23876685.1**

(22) Date of filing: **10.10.2023**

(51) International Patent Classification (IPC):
***C07C 231/02*** (2006.01) ***C07C 235/08*** (2006.01)
***A61K 8/42*** (2006.01) ***A61K 31/231*** (2006.01)
***A61P 29/00*** (2006.01) ***A61P 39/00*** (2006.01)
***A61P 39/06*** (2006.01) ***A61Q 19/00*** (2006.01)
***A61Q 19/08*** (2006.01) ***A23L 33/10*** (2016.01)

(52) Cooperative Patent Classification (CPC):
**A23L 33/10; A61K 8/42; A61K 31/231; A61P 29/00; A61P 39/00; A61P 39/06; A61Q 19/00; A61Q 19/08; C07C 231/02; C07C 235/08;** Y02P 20/55

(86) International application number:
**PCT/CN2023/123721**

(87) International publication number:
**WO 2024/078483 (18.04.2024 Gazette 2024/16)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **10.10.2022 CN 202211238109**

(71) Applicant: **Shenzhen Dikeman Biotechnology Co., Ltd.**
**Shenzhen, Guangdong 518000 (CN)**

(72) Inventors:
- **YANG, Chaowen**
  **Shenzhen, Guangdong 518000 (CN)**
- **YE, Liu**
  **Shenzhen, Guangdong 518000 (CN)**

(74) Representative: **Global IP Europe Patentanwaltskanzlei**
**Pfarrstraße 14**
**80538 München (DE)**

(54) **NOVEL CERAMIDE, PREPARATION METHOD THEREFOR, AND USE THEREOF**

(57) The present disclosure belongs to the technical field of biological medicines. Disclosed is a novel ceramide which has a structure represented as formula I

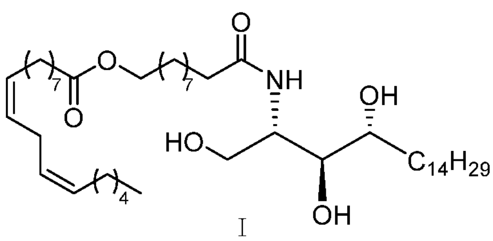

I

Further disclosed in the present disclosure are a preparation method for and the use of the ceramide. The present disclosure uses naturally-sourced and readily-available 10-hydroxycarboxylic acid as a starting raw material to construct the ceramide having a novel structure. The preparation method has a simple and convenient synthetic route and is suitable for large-scale production. Meanwhile, the obtained ceramide shows more excellent effects in the aspects of tissue repair, anti-inflammatory repair, tissue healing, moisturizing and anti-aging, and thus can be used in the fields of health care products, cosmetics and drugs.

EP 4 603 476 A1

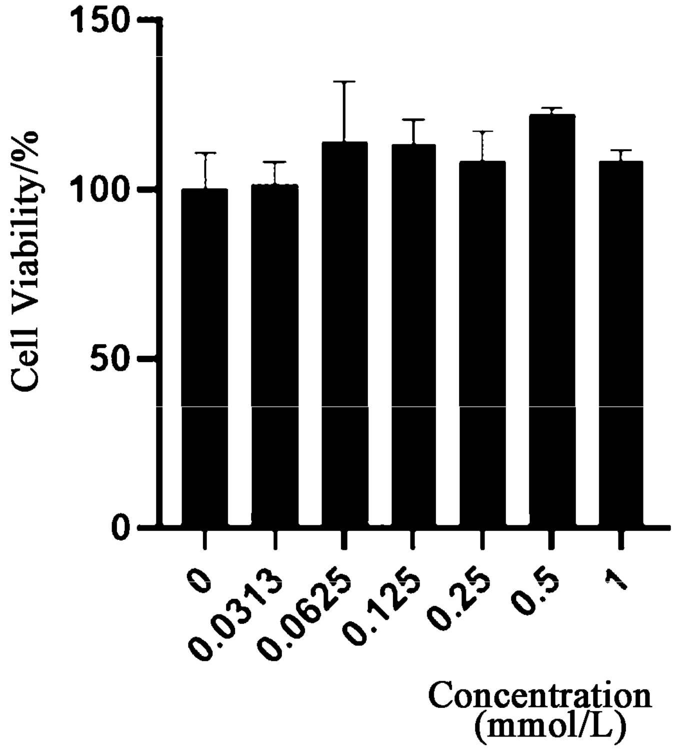
150
100
50
0
Cell Viability/%
0
0.0313
0.0625
0.125
0.25
0.5
1
Concentration
(mmol/L)

FIG. 1

## Description

### TECHNICAL FIELD

The present disclosure relates to a field of biological medicine, in particular, to a novel ceramide and preparation method and application thereof.

### BACKGROUND ART

Ceramide (also known as molecular anchor) naturally occurs in skin and is a very important component of skin barrier (i.e., stratum corneum), with a content of up to 40 to 50 wt%. In terms of chemical structure, ceramide is a sphingosine lipid composed of sphingosine-like long-chain bases and fatty acids, of which the carbon chain length, degree of unsaturation and number of hydroxyl groups of the sphingosine part and fatty acid part can be varied, and therefore, the ceramide molecule is not unique and refers to a class of compounds.

Ceramides are especially widespread in the skin. There are 9 kinds of natural ceramides as currently-known, and various kinds of ceramides are used in skin care products. Ceramides exhibit excellent properties in regulating skin barrier function, restoring skin moisture and enhancing adhesion among skin keratinocytes.

A decrease in an amount of ceramides will lead to xerosis of the skin impairing the skin's surface defense function, and making it easier for foreign substances to invade and cause secondary infection of the skin, thus causing skin rejection. Specifically, the invasive substances cause cytokines to be released from cells such as keratinocytes, Langerhans cells, and melanin cells of epidermal cells, thereby causing inflammation and the like. Therefore, in order to maintain and improve the skin barrier, the moisturizing function on skin is important, and a mixture of physiological lipids containing ceramide compounds can promote recovery of impaired skin barrier function compared to common moisturizers. Results of clinical trials indicate that the mixture of physiological lipids exhibits similar effects to those of topical steroid formulations having a moderate-to-high efficacy in improving symptoms of patients suffering from atopic dermatitis.

Due to the importance of the ceramides, many cosmetics and pharmaceuticals companies are researching and developing corresponding products. However, due to facts that natural ceramides are not easy for extraction, cost-expensive and thus unsuitable for commercialization, it is difficult to produce the natural ceramides on a large scale. Some companies are making efforts to develop ceramides that are structurally similar to those found in the skin and can functionally provide the same effect.

Therefore, considering a wide demand for functional ceramides in the market, it is very necessary to rapidly synthesize ceramides by using short-chain fragments that are naturally sourced and easily available, to solve the problem of insufficient supply of ceramides and enhance their efficacy.

### SUMMARY

An object of the present disclosure is to provide a ceramide having a novel structure.

Another object of the present disclosure is to provide a method for synthesis of the ceramide using readily-available 10-hydroxydecanoic acid as a starting material.

A further object of the present disclosure is to provide use of the ceramide.

In order to achieve one of the above objects, the present application adopts technical solutions as follows.

A ceramide has a structure of formula I, or enantiomers or diastereoisomers thereof:

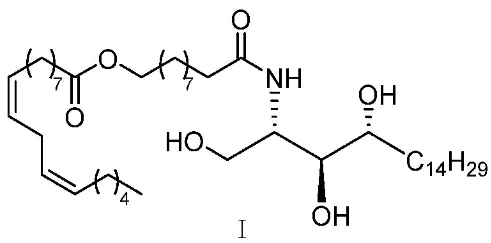

I

A preparation method for the ceramide comprises the following steps of:

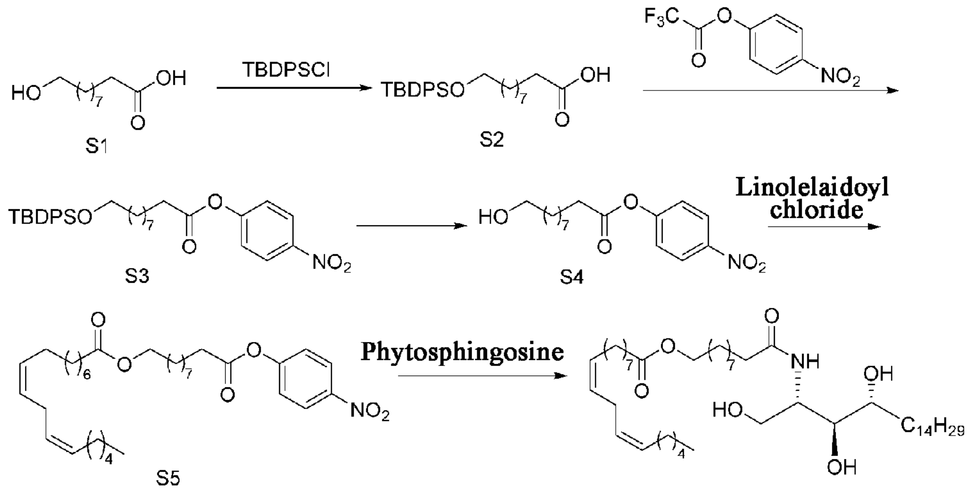


obtaining a compound S2 from reaction of a compound S1 with TBDPSC1;

obtaining a compound S3 from reaction of the compound S2 with 4-nitrophenyl trifluoroacetate;

obtaining a compound S4 by deprotection of the compound S3;

obtaining a compound S5 from reaction of the compound S4 with linolelaidoyl chloride;

obtaining the ceramide from reaction of the compound S5 with phytosphingosine.

Further, a molar ratio of the compound S1 to TBDPSC1 is 1: (1 to 3).

Further, a molar ratio of the compound S2 to 4-nitrophenyl trifluoroacetate is 1: (1 to 3).

Further, a molar ratio of the compound S4 to linolelaidoyl chloride is 1: (1 to 3).

Further, a molar ratio of the compound S5 to phytosphingosine is 1: (1 to 2).

The ceramides can be used in the field of cosmetics, health products or pharmaceuticals.

According to a first aspect, the ceramide has tissue-repairing effects. The cell viability is more than 114% when the concentration of ceramide is 0.0625 mM, and the cell viability is more 120% when the concentration is 0.5 mM.

According to a second aspect, the ceramide has anti-inflammatory repairing effects, the principle is that it inhibits the expression of interleukin-6 (IL-6) cytokine. The inhibition rate on cytokine IL-6 is above 46% when the ceramide is at a concentration of 0.1 nM; the inhibition rate on cytokine IL-6 is above 73% when the ceramide is at a concentration of 0.2 mM; the inhibition rate on cytokine IL-6 is above 86% when the ceramide is at a concentration of 0.4 mM; and the inhibition rate on cytokine IL-6 is above 97% when the ceramide is at a concentration of 1.0 mM. The anti-inflammatory repairing effects represent a unique efficacy discovered by the inventors through studies on biological activities of the ceramide. To date, no existing ceramide products have been reported to have such anti-inflammatory and reparative efficacy.

According to a third aspect, the ceramide has tissue-healing effects. When the ceramide is at a concentration of 0. 08 mM, the cell healing rate is more than 74%.

According to a fourth aspect, the ceramide has moisturizing effects, the principle is that it increases the expression of AQP3 (aquaporin 3) aquaporin protein. The expression level of AQP3 is more than 112% relative to a solvent control group when the ceramide is at a concentration of 0.4 mM; the expression of AQP3 is more than 133% relative to a solvent control group when the ceramide is at a concentration of 0.8 mM; and the expression of AQP3 is more than 147% relative to a solvent control group when the ceramide is at a concentration of 1.6 mM.

According to a fifth aspect, the ceramide has anti-aging effects, the principle is that it improves the inhibition rate on elastase. The inhibition rate on the elastase is above 18% when the ceramide is at a concentration of 0.4 mM; the inhibition rate on the elastase is above 22% when the ceramide is at a concentration of 0.8 mM; and the inhibition rate on the elastase is above 25% when the ceramide is at a concentration of 1.0 mM. The anti-aging properties represent a unique efficacy discovered by the inventors through studies on the biological activities of the ceramide. To date, no existing ceramide products have been reported to have such anti-aging efficacy.

A composition comprises the ceramide, isomers, pharmaceutically acceptable salts, hydrates or solvates thereof, as an active ingredient. The composition has effects in tissue repairing, anti-inflammatory repairing, tissue healing, moisturizing or anti-aging.

A "pharmaceutically acceptable salt" as used herein means a salt of a compound according to one aspect of the

disclosure that is pharmaceutically acceptable and exhibits desired pharmacological activities of the parent compound. Such salts include: (1) acid addition salts formed with the following acids: inorganic acids, such as hydrochloric acid, hydrobromic acid, sulfuric acid, nitric acid, phosphoric acid, etc.; and organic acids, such as acetic acid, propionic acid, hexanoic acid, cyclopentylpropionic acid, glycolic acid, pyruvic acid, lactic acid, propanedioic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2-naphthalene sulfonic acid, p-toluenesulfonic acid, camphorsulfonic acid, 4-methylbicyclo[2,2,2]oct-2-ene-1-carboxylic acid, glucoheptonic acid, hydrocinnamic acid, trimethylacetic acid, tert-butylacetic acid, docecyl sulfonic acid, gluconic acid, glutamate, hydroxyl naphthalene carboxylic acid, salicyclic acid, stearic acid, and muconic acid; or (2) a salt produced from substitution of an acidic proton present in the parent compound.

A "hydrate" as used herein means a compound that binds to water. The binding between the compound and water includes non-covalent binding.

A "solvate" as used herein means a complex formed by a solute molecule or ion with a solvent molecule or ion.

An "isomer" as used herein means that a compound of the present disclosure or a salt thereof has the same chemical or molecular formula but has different optical or steric properties.

Unless otherwise specified, the term "a compound according to the present disclosure" or "ceramide" includes a compound itself, pharmaceutically acceptable salts, hydrates, solvates, isomers thereof.

The present disclosure has the following beneficial effects:

The present disclosure synthesizes a ceramide with a novel structure by using naturally-sourced, commercially-available, and cost-effective 10-hydroxycarboxylic acid as a starting material, and therefore avoiding use of raw materials that are difficult to be commercially available. The synthetic route is simple and convenient, which is suitable for scale-up production. The resulting ceramide has excellent efficacy in tissue repairing, anti-inflammatory repairing, tissue healing, moisturizing and anti-aging, and exhibits better effects than those of known ceramide 3 and ceramide 3. In particular, the unique efficacy of anti-inflammation and anti-aging of such compounds has been rarely reported, and the resulting ceramide can be used in the fields of health products, cosmetics and pharmaceuticals.

## BRIEF DESCRIPTION OF DRAWINGS

FIG. 1 is a bar chart illustrating the results of cell proliferation and viability assay for the ceramide according to the present disclosure;

FIG. 2 is a bar chart illustrating the results of cell proliferation and viability assay for ceramide 3;

FIG. 3 is a bar chart illustrating the results of cell proliferation and viability assay for ceramide 3B;

FIG. 4 is a bar chart illustrating the results of anti-inflammatory repair efficacy assay for the ceramide according to the present disclosure;

FIG. 5 is a bar chart illustrating the results of anti-inflammatory repair efficacy assay for different ceramides in Example 3;

FIG. 6 is a bar chart illustrating the results of tissue healing ability assay for different ceramides in Example 4;

FIG. 7 is a bar chart illustrating the results of moisturizing efficacy assay for the ceramide according to the present disclosure;

FIG. 8 is a bar chart illustrating the results of moisturizing efficacy assay for different ceramides in Example 5;

FIG. 9 is a bar chart illustrating the results of anti-aging efficacy assay for the ceramide according to the present disclosure;

FIG. 10 is a bar chart illustrating the results of anti-aging efficacy assay for different ceramides in Example 6.

## DETAILED DESCRIPTION

The present disclosure will be further described below with reference to specific embodiments.

All reactions were carried out under nitrogen atmosphere. Chemicals were purchased as commercial products and were not further purified unless otherwise specified. Dichloromethane, tetrahydrofuran, pyridine and N, N-dimethylformamide as used in the experiments were all anhydrous solvents. Thin-layer chromatography (TLC) was performed using a 60F254 silica gel plate. Silica gel (with a particle size of 0.040-0.063 mm) commercially available from Qingdao Haiyang Chemical Co., Ltd (Qingdao, Shandong, China) was used for silica gel column chromatography. Ultraviolet (UV) light (254 nm) or iodine was used for TCL visualization. Nuclear magnetic resonance (NMR) spectrum was characterized using a Bruker DPX 400 nuclear magnetic resonance spectrometer, with $^1$H NMR of 400 MHz, using deuterated methanol, deuterated dimethyl sulfoxide (DMSO) or deuterated tetrahydrofuran as a solvent, and using tetramethylsilane (TMS) as an internal standard. Chemical shift is expressed in parts per million (ppm) and coupling constant is expressed in a Hz. In $^1$H NMR, $\delta$ represents a chemical shift; s represents singlet; d represents doublet; t represents triplet; q represents quartet; and m represents multiplet.

EA denotes ethyl acetate; DCM denotes dichloromethane; DMF denotes N, N-dimethylformamide; Py. denotes pyridine; Im. denotes imidazole; TBDPSC1 denotes t-butylchlorodiphenylsilane; and TBAF denotes tetrabutylammonium fluoride.

Example 1

Synthesis of ceramides.

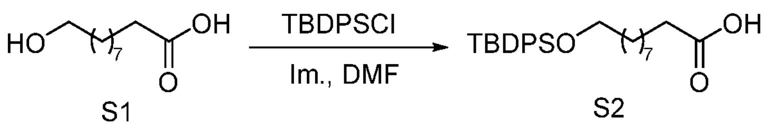


S1 S2

1.0 equivalent (eq, 160 mmol) of S1 and 2.0 eq (320 mmol) of imidazole were dissolved in 100 mL of DMF, and cooled in an ice bath, with dropwise addition of a mixture solution of 2.0 eq (320 mmol) of TBDPSC1 and 50 mL of DMF, and after the dropwise addition, the mixture was heated to 40 °C and reacted until the reaction of the starting material S1 was completed as detected by TLC to obtain a reaction product.

Post-treatment: The reaction product was diluted with 400 mL of EA, washed twice with 250 mL of saturated solution of $NH_4Cl$ and then twice with 300 mL of saturated solution of NaCl, and then anhydrous $Na_2SO_4$ was added into the organic phase, followed by drying, filtering and concentrating in vacuum. The resulting residue was then purified by silica gel column chromatography to obtain 79.4 g of a product S2.

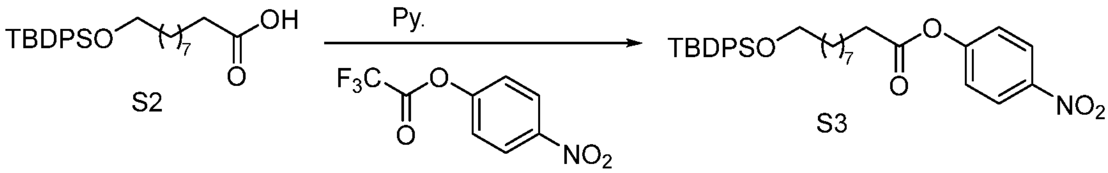


S2 S3

1.0 eq (160 mmol) of S2 and 1.0 eq (160 mmol) of 4-nitrophenyl trifluoroacetate were dissolved in 150 mL of pyridine and stirred at room temperature until the reaction of S2 was completed as detected by TLC to obtain a reaction product.

Post-treatment: The reaction product was diluted with 300 mL of EA, washed twice with 150 mL of dilute hydrochloric acid and twice with 300 mL of water followed by once with 200 mL of saturated solution of NaCl, and then anhydrous $Na_2SO_4$ was added into the organic phase, followed by drying, filtering, and concentrating in vacuum to obtain an oily product. The oily product was then purified by silica gel column chromatography to obtain 67.4 g of a product S3.

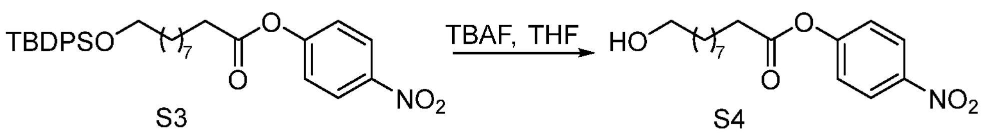


S3 S4

1.0 eq (124 mmol) of S3 was dissolved in 100 mL of tetrahydrofuran (THF), and cooled in an ice bath, with dropwise addition of 1.2 eq (149 mmol) of tetrabutylammonium fluoride (TBAF) dissolved in 40 mL of THF. After the dropwise addition, the reaction was carried out at room temperature until the reaction of S3 was completed as detected by TLC to obtain a reaction product.

Post-treatment: The reaction product was diluted with 200 mL of EA, and washed once with 100 mL of dilute hydrochloric acid and once with 100 mL of saturated solution of NaCl, and then anhydrous $Na_2SO_4$ was added into the organic phase, followed by drying, filtering, and concentrating in vacuum to obtain an oily product. The oily product was then purified by silica gel column chromatography to obtain 21.6 g of a product S4.

$^1H$ NMR (400 MHz, Methanol-$d_4$) δ 8.34 - 8.23 (m, 2H), 7.39 - 7.30 (m, 2H), 3.54 (t, $J$ = 6.6 Hz, 2H), 2.63 (t, $J$ = 7.4 Hz, 2H), 1.73 (t, $J$ = 6.8 Hz, 2H), 1.53 (t, $J$ = 6.8 Hz, 2H), 1.46 - 1.26 (m, 10H).

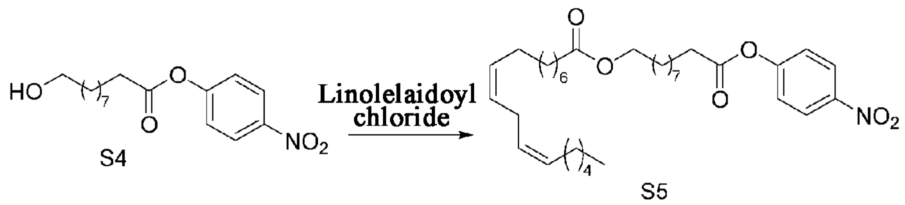


1.0 eq (71 mmol) of S4 was dissolved in 200 mL of pyridine, and cooled in an ice bath, with dropwise addition of 4.0 eq (284 mmol) of linolelaidoyl chloride. After the dropwise addition, the reaction was carried out at room temperature until the reaction of S4 was completed as detected by TLC to obtain a reaction product.

Post-treatment: The reaction product was diluted with 400 mL of DCM, and washed twice with 600 mL of dilute hydrochloric acid and twice with 500 mL of saturated solution of NaCl, and then anhydrous $Na_2SO_4$ was added into the organic phase, followed by spin-drying to obtain a black oily product. The black oily product was then purified by silica gel column chromatography to obtain 30 g of a product S5.

$^1H$ NMR (400 MHz, Methanol-$d_4$) δ 8.32 - 8.25 (m, 2H), 7.37 - 7.29 (m, 2H), 5.41 - 5.31 (m, 4H), 3.64 (t, $J$ = 6.6 Hz, 2H), 2.53 (t, $J$ = 7.4 Hz, 2H), 2.29 (t, $J$ = 7.6 Hz, 2H), 2.26 - 2.19 (m, 4H), 2.08 (q, $J$ = 6.9 Hz, 4H), 1.63 (t, $J$ = 6.8 Hz, 4H), 1.43 (t, $J$ = 6.8 Hz, 4H), 1.46 - 1.26 (m, 20H), 0.86 (t, $J$ = 6.9 Hz, 3H).

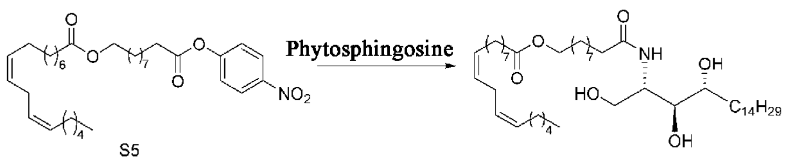


1.0 eq (53 mmol) of S5 and 1.0 eq (53 mmol) of phytosphingosine were dissolved in 100 mL of pyridine, and then heated to 45 °C and reacted until the reaction of phytosphingosine was completed as detected by TLC to obtain a reaction product.

Post-treatment: The reaction product was diluted with 200 mL of THF, washed twice with 200 mL of dilute hydrochloric acid and twice with 200 mL of saturated solution of NaCl, and then anhydrous $Na_2SO_4$ was added into the organic phase, followed by spin-drying to obtain a crude product. The crude product was then purified by silica gel column chromatography to obtain 15 g of a product.

$^1H$ NMR (400 MHz, Chloroform-d) δ 6.37 (d, $J$ = 7.5 Hz, 1H), 5.36 (tddt, $J$ = 10.7, 5.4, 3.7, 1.6 Hz, 4H), 4.15 (dt, $J$ = 7.2, 2.6 Hz, 1H), 4.05 (t, $J$ = 6.8 Hz, 2H), 3.91 (t, $J$ = 6.8 Hz, 2H), 3.73 (s, 2H), 3.67 - 3.51 (m, 2H), 2.88 - 2.70 (m, 3H), 2.29 (t, $J$ = 7.6 Hz, 2H), 2.26 - 2.19 (m, 2H), 2.05 (q, $J$ = 6.9 Hz, 4H), 1.80 - 1.70 (m, 1H), 1.61 (p, $J$ = 6.9 Hz, 6H), 1.55 - 1.44 (m, 2H), 1.39 - 1.21 (m, 47H), 0.88 (td, $J$ = 6.9, 4.0 Hz, 6H).

Example 2

Cell proliferation and viability as detected by methylthiazolyldiphenyl-tetrazolium bromide (MTT) assay: human keratinocytes (HaCaT) cells were seed in a 96-well plate at a cell density of $1 \times 10^4$ cells/well and placed in an incubator overnight. After 24 hours of incubation, the supernatant was discarded and 100 μL of culture medium containing samples (i.e., the product obtained in Example 1) at different concentrations or blank control (culture medium only) was added into the cells, and incubated for additional 24 hours, followed by removing the culture medium, adding 100 μL of MTT to each well, measuring the absorbance at 450 nm, and calculating the cell viability as: Cell viability = $A_{dosing}/A_{blank} \times 100\%$.

The results are shown in FIG. 1, the compound demonstrated a cell viability of 114.7% at a concentration of 0.0625 mM, and a cell viability of 122.3% at a concentration of 0.5 mM, indicating an evident effect of promoting cell

proliferation and tissue-repairing potential. Meanwhile, the compound exhibits no cytotoxicity and a relatively good biological safety at a concentration of up to 1mM.

The results of cell proliferation and viability assay for ceramide 3 and ceramide 3B were shown in FIGs. 2 and 3, the ceramide according to the present disclosure and ceramide 3B exhibit relatively good abilities of cell healing and tissue-repairing.

Example 3

Anti-inflammatory repair efficacy as detected by Lipopolysaccharide(LPS)-induced cell assay

B16 murine melanoma cells were seeded in a 96-well plate at a cell density of $1 \times 10^4$ cells/well and placed in an incubator overnight to allow cell adhesion. After 24 hours of incubation, the supernatant was discarded, and 100 μL of samples (i.e., the product obtained in Example 1) at different concentrations diluted with DMEM medium and DMEM culture medium free of the samples as a negative control group were added into the cells, each group in triplicate, followed by incubating under an atmosphere of 5% $CO_2$ expressed by mass fraction at 37 °C. At 2 hours after administration, 10 μg/mL LPS was added into both lipopolysaccharide model groups and experimental groups and then co-incubated for 24 hours. After the reaction, 50 μL of the cell supernatant was taken and detected for IL-6 gene expression using a IL-6 ELISA kit.

The results are shown in FIG. 4, indicating that models of inflammation of cells were successfully established using LSP, and expression level of IL-6 inflammatory factors in the model cells was up to 23.14 times of that in the control group. The results demonstrate that the compound according to the present disclosure has an excellent effect of inhibiting the expression of the IL-6 cytokine. When the compound was at a concentration of 0.1, 0.2, 0.4, 0.8 or 1.00 mM, the expression level of IL-6 factor was 12.37, 6.15, 3.04, 1.31 or 0.65 times relative to that in the control group, respectively; and compared with the LPS-induced model group, the IL-6 expression was inhibited by 46.54%, 73.42%, 86.86%, 94.30% or 97.19%, respectively. Therefore, the compounds have good anti-inflammatory effect and are expected to have promising potential for promoting the repair of inflammation-damaged skin.

Results of comparison among 1 mmol/L of ceramide 3, ceramide 3B and the ceramide according to the present disclosure were shown in FIG. 5, indicating that all the three ceramides effectively inhibit the expression of IL-6 inflammatory factors, and the ceramide according to the present disclosure exhibits the most significant inhibitory effect on IL-6 inflammatory factors.

Example 4

Ability of tissue healing as detected by scratch assay

Principle: as the cells grow to be a confluent monolayer, a blank area is created on the confluent monolayer using a scratching tool, i.e., removing the cells within the blank area by mechanical force, and then migration of the cells towards the cell-free blank area is observed after a period of incubation, and the migration ability of the cells is reflected by measurement of their migration distance.

Procedure are as follows.

1. Drawing lines on culture plate: first, a Marker pen was used to drawing transverse lines on the back of a 6-well plate with a uniform gap of 0.5 to 1 cm through a ruler, with the transverse lines across the wells and each well is traversed by at least 5 lines. Note that the drawn lines should not be too thick.2. Seeding cells: approximately $5 \times 10^5$ cells were seeded into the well (the number of cells were varied depending on of the cell type, and can be adjusted according to the grow rate of the cell). The principle for seeding was to adjust the density of seeded cells to ensure 100% confluency after overnight incubation.

3. Cell scratching: on the second day, a pipette tip, perpendicular to cell plane, was used to scratch on the layer of cells along the line drawn on the back of the plate on the first day (it is preferred to use the same pipette tip for different wells).

4. Washing cells: after the scratching is completed, the cells were washed three times with sterile phosphate-buffered saline (PBS) to wash off non-adherent cells, i.e. cells that fell off during scratching, and the gaps left by scratching were clearly visible, and then was replaced with a fresh serum-free medium.

5. Cell incubation and observation: samples (the compound of Example 1, ceramide 3, and ceramide 3B) were diluted with a medium (at a concentration of 0.08 mM) and then added to a cell culture dish, and the cells were then placed in an incubator (37 °C, 5 wt% $CO_2$) for incubation. After 24 hours of incubation, the cells were taken out, observed with a microscope, and measured for the width of the scratch, followed by photographing and calculating the healing rate with Image J software.

The results are shown in FIG. 6, indicating that the self-healing rate of the solvent control group after 24 hours was 35.74%, and the healing rate of the ceramide according to the present disclosure after 24 hours was 74.48%, having a better ability of tissue healing than ceramide 3 and ceramide 3B. Obviously, the compound according to the present disclosure increases the cell healing rate and have good activity in skin tissue repairing.

Example 5

Moisturizing efficacy as detected by AQP3 cell assay

Aquaporin 3 (AQP3) is a transporter factor responsible for transport of water, glycerol and urea on cell membrane, which is mainly expressed in keratinocytes and skin fibroblasts. AQP3 is not only involved in skin hydration and barrier function, but also plays an important role in skin damage, repair and healing, and is an important guarantee for maintenance of normal skin morphology and function.

The HaCat cells were seeded in a 96-well plate at a density of $1 \times 10^4$ cells/well and placed in an incubator overnight to allow cell adhesion. After 24 hours of incubation, the supernatant was discarded, and 100 μL of samples (i.e., the product obtained in Example 1) at different concentrations diluted with DMEM medium and DMEM medium free of the samples as a negative control group were added into the plate, each group in triplicate, followed by incubating under an atmosphere of 5% $CO_2$ expressed by mass fraction at 37 °C. At 2 hours after administration, 10 μg/mL LPS was added into both lipopolysaccharide model groups and experimental groups and then co-incubated for 24 hours. After completing the reaction, 50 μL of the cell supernatant was taken and detected for the AQP3 gene expression in the cells using a AQP3 assay kit.

The results are shown in FIG. 7, indicating that the compound according to the present disclosure effectively increased the expression level of AQP3 in a concentration-dependent manner. When the compound was at a concentration of 0.4, 0.8 or 1.6 mM, the expression level of AQP3 was 1.12, 1.33 or 1.47 times relative to the solvent control group, respectively.

Ceramide 3 and ceramide 3B at a concentration of 2.5mmol/L were compared with the ceramide according to the present disclosure, the results are shown in FIG. 8, indicating that all the three ceramides effectively increase the expression level of AQP3, and the ceramide according to the present disclosure has the most significant effect and exhibits the best moisturizing performance.

Example 6

Anti-aging efficacy as detected by elastase inhibition method

2 mL of 2 mg/mL elastase solution was taken and added into samples at different concentrations and then vortex-mixed sufficiently and homogenously, followed by shaking using a shaker at 37 °C with a rotation rate of 400 r/min for 20 min. 5 mL of 0.5 mol/L phosphate buffer at pH 6.0 was immediately added, and vortex-mixed homogenously, followed by taking the homogenously-mixed solution (quantum satis) into a 2 mL centrifuge tube, and centrifuging at 9391 × g for 10 min, and 200 μL of the supernatant was pipetted precisely into a 96-well plate, and measured for absorbance with a microplate reader at 495 nm, and scanned spectrum ranging from 400 to 800 nm.

A substrate plus enzyme solution was used as a blank control group, and the substrate plus enzyme solution and sample solution (the product obtained in Example 1) was used as an enzyme inhibition group, and the substrate plus the sample solution without the enzyme solution was used as a background, each group in triplicate. Inhibition rate (%) = [1-(An-An')/(A0-A0')] × 100%, where A0 denotes absorbance of the enzyme solution without the sample, and A0' denotes absorbance of the substrate only without sample and enzyme, and An denotes absorbance of the sample solution only, and An' denotes absorbance of the sample solution without enzyme. If An' > An, a promotion effect is exhibited, and calculated as

$$\text{Promotion rate (\%)} = [1-(An'-An)/(A0-A0')] \times 100\%.$$

The results are shown in FIG. 9, indicating that the compound according to the present disclosure effectively increases the elastase inhibition rate in a concentration-dependent manner. When the compound was at a concentration of 0.4, 0.8, or 1.0 nM, the inhibition rates was 18.21%, 22.07% or 25.37%, respectively., indicating that the compound can inhibit the expression of elastase, protect elastin, and maintain or improve skin elasticity.

of Ceramide 3 and Ceramide 3B at a concentration of 1 mmol/L were compared with the ceramide according to the present disclosure, the results were shown in FIG. 10, indicating that the effects of the ceramide according to the present application were much better than those of ceramide 3 and ceramide 3B, and exhibits the best effect of elastase inhibition.

The present disclosure comprehensively tested the efficacy of the ceramides in cell repairing, proliferation, anti-inflammation, moisturizing, and elastase inhibition. Compared with commercially available ceramide 3 and ceramide 3B, the ceramide according to the present disclosure demonstrates the most excellent and comprehensive performance, especially in anti-inflammatory, tissue healing and elastase inhibition.

The foregoing descriptions represent merely specific embodiments of the present disclosure, but the scope of protection of the present disclosure is not limited thereto. Any variations or substitutions that can be easily envisaged by those skilled in the art within the technical scope disclosed in the present disclosure shall be encompassed in the scope of protection of the present disclosure. Therefore, the scope of protection of the present disclosure should be based on the scope of protection of the claims.

## Claims

1. A ceramide having a structure of formula I, or enantiomers or diastereoisomers thereof:

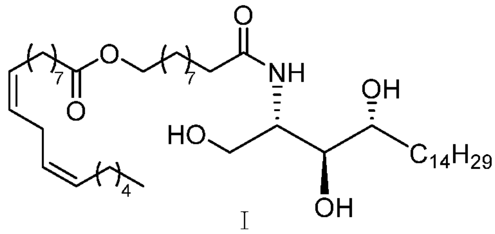

I

2. A preparation method for the ceramide according to claim 1, comprising the steps of:

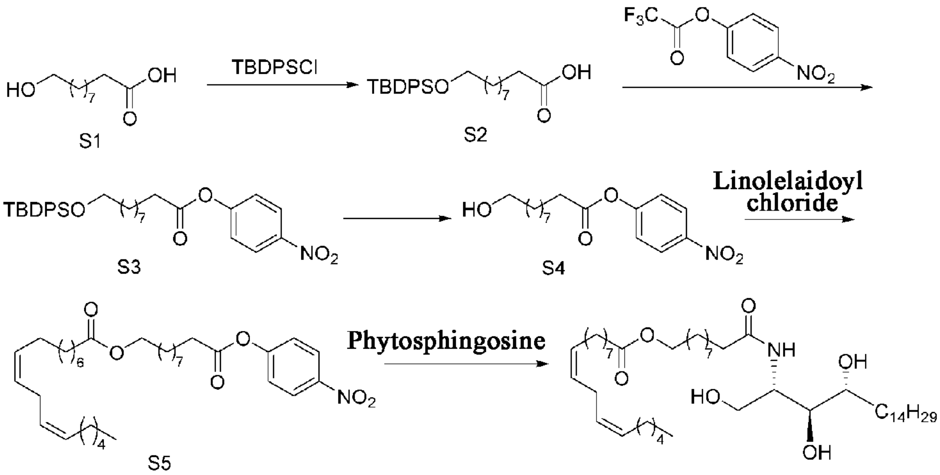


obtaining a compound S2 from reaction of a compound S1 with TBDPSC1;
obtaining a compound S3 from reaction of the compound S2 with 4-nitrophenyl trifluoroacetate;
obtaining a compound S4 by deprotection of the compound S3;
obtaining a compound S5 from reaction of the compound S4 with linolelaidoyl chloride;
obtaining the ceramide from reaction of the compound S5 with phytosphingosine.

3. The method according to claim 2, wherein a molar ratio of the compound S1 to TBDPSC1 is 1: (1 to 3); a molar ratio of the compound S2 to 4-nitrophenyl trifluoroacetate is 1: (1 to 3); a molar ratio of the compound S4 to the linolelaidoyl chloride is 1: (1 to 3); and a molar ratio of the compound S5 to the phytosphingosine is 1: (1 to 2).

4. Use of the ceramide according to claim 1 in cosmetics, health products or pharmaceuticals.

5. The use according to claim 4, wherein the ceramide has tissue-repairing effects; and the cell viability is more than 114% when the ceramide is at a concentration of 0.0625 mM, and/or the cell viability is more than 120% when at a concentration of 0.5 mM.

6. The use according to claim 4, wherein the ceramide has anti-inflammatory repair effects; the inhibition rate on cytokine IL-6 is above 46% when the ceramide is at a concentration of 0.1 mM, and/or the inhibition rate on cytokine IL-6 is above 73% when the ceramide is at a concentration of 0.2 mM, and/or the inhibition rate on cytokine IL-6 is above 86% when the ceramide is at a concentration of 0.4 mM, and/or the inhibition rate on cytokine IL-6 is above 94% when the ceramide is at a concentration of 0.8 mM, and/or the inhibition rate on cytokine IL-6 is above 97% when the ceramide is at a concentration of 1.0 mM.

7. The use according to claim 4, wherein the ceramide has tissue-healing effects; and the cell healing rate is more than 74% when the ceramide is at a concentration of 0.08 mM.

8. The use according to claim 4, wherein the ceramide has moisturizing effects; the expression level of AQP3 is more than 112% relative to a solvent control group when the ceramide is at a concentration of ceramide of 0.4 mM; the expression level of AQP3 is more than 133% relative to a solvent control group when the ceramide is at a concentration of ceramide of 0.8 mM; the expression level of AQP3 is more than 147% relative to a solvent control group when the ceramide is at a concentration of ceramide of 1.6 mM.

9. The use according to claim 4, wherein the ceramide hass anti-aging effects; the inhibition rate on elastase is above 18% when the ceramide is at a concentration of 0.4 mM, and the inhibition rate on elastase is above 22% when the ceramide is at a concentration of 0.8 mM, and the inhibition rate on elastase is above 25% when the ceramide is at a concentration of 1.0 mM.

10. A composition comprising the ceramide according to claim 1, isomers, pharmaceutically acceptable salts, hydrates or solvates thereof as an active ingredient.

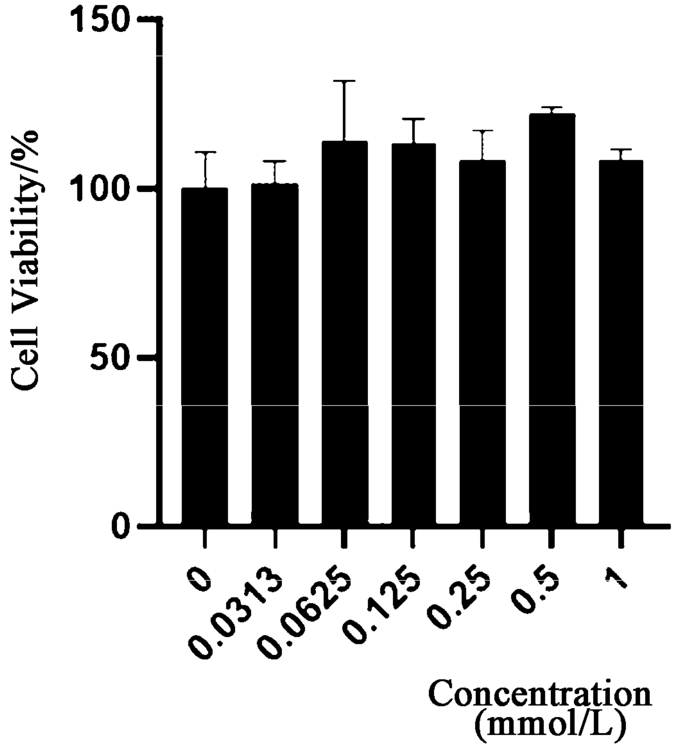
150
100
50
0
Cell Viability/%
0
0.0313
0.0625
0.125
0.25
0.5
1
Concentration (mmol/L)

FIG. 1

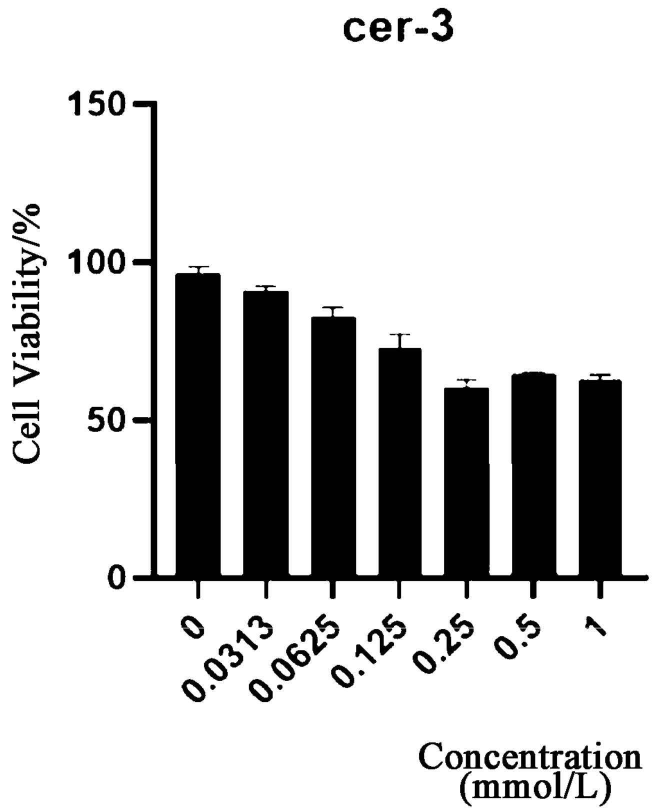
cer-3
150
100
50
0
Cell Viability/%
0
0.0313
0.0625
0.125
0.25
0.5
1
Concentration (mmol/L)

FIG. 2

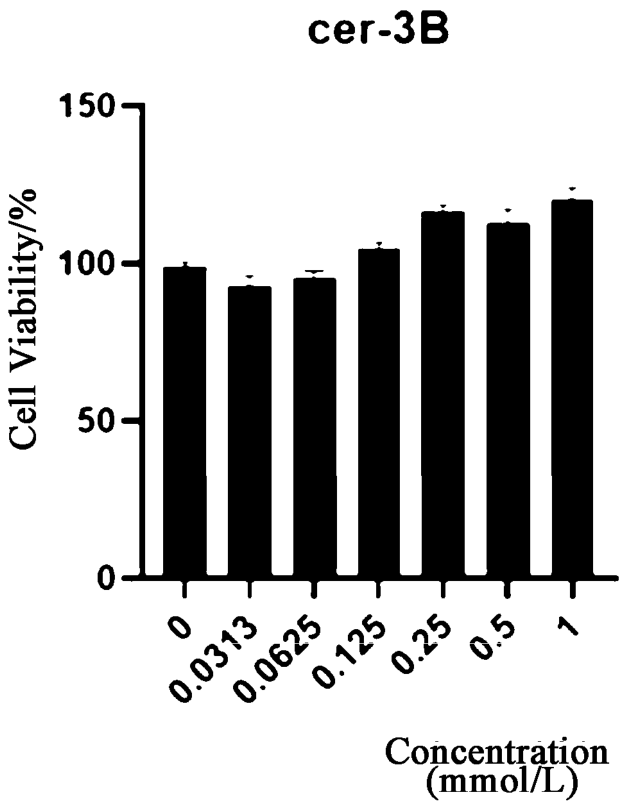
cer-3B
150
100
50
0
Cell Viability/%
0
0.0313
0.0625
0.125
0.25
0.5
1
Concentration
(mmol/L)

FIG. 3

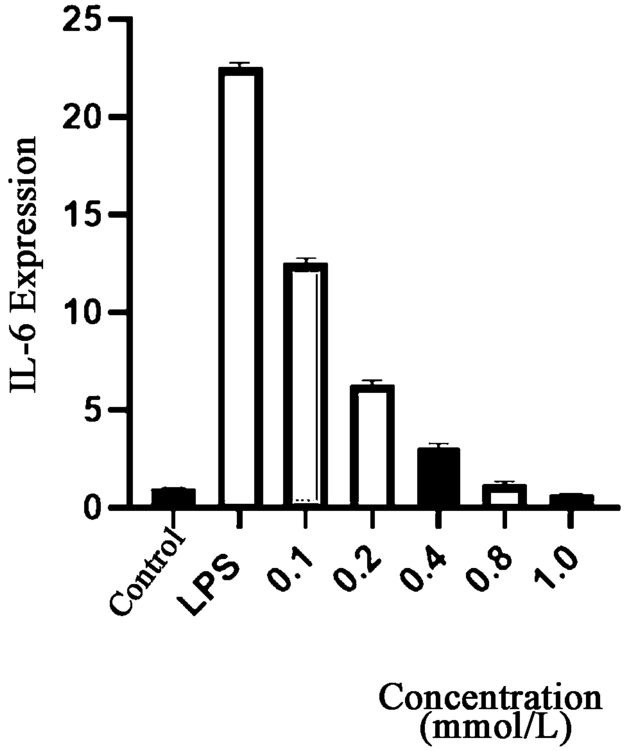
25
20
15
10
5
0
IL-6 Expression
Control
LPS
0.1
0.2
0.4
0.8
1.0
Concentration
(mmol/L)

FIG. 4

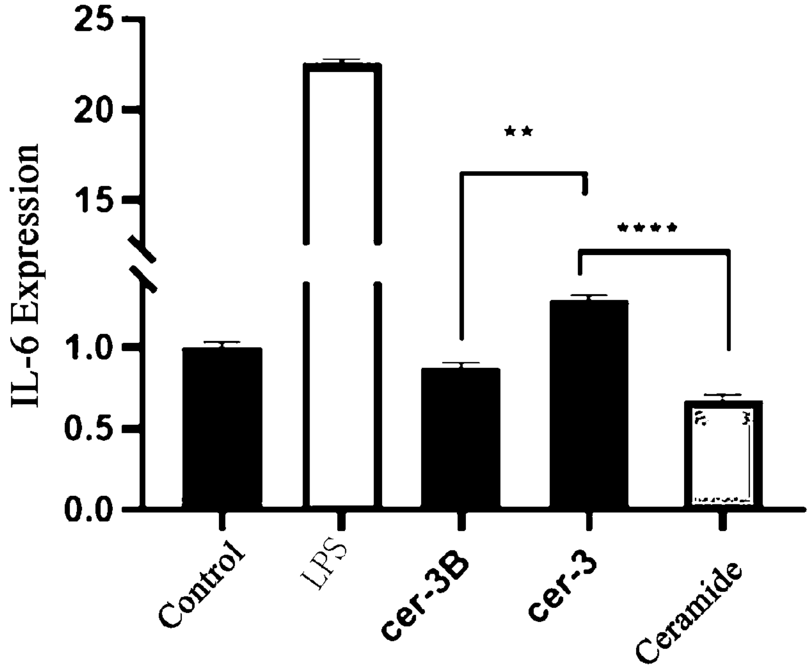
25
20
15
1.0
0.5
0.0
IL-6 Expression
**
****
Control
LPS
cer-3B
cer-3
Ceramide

FIG .5

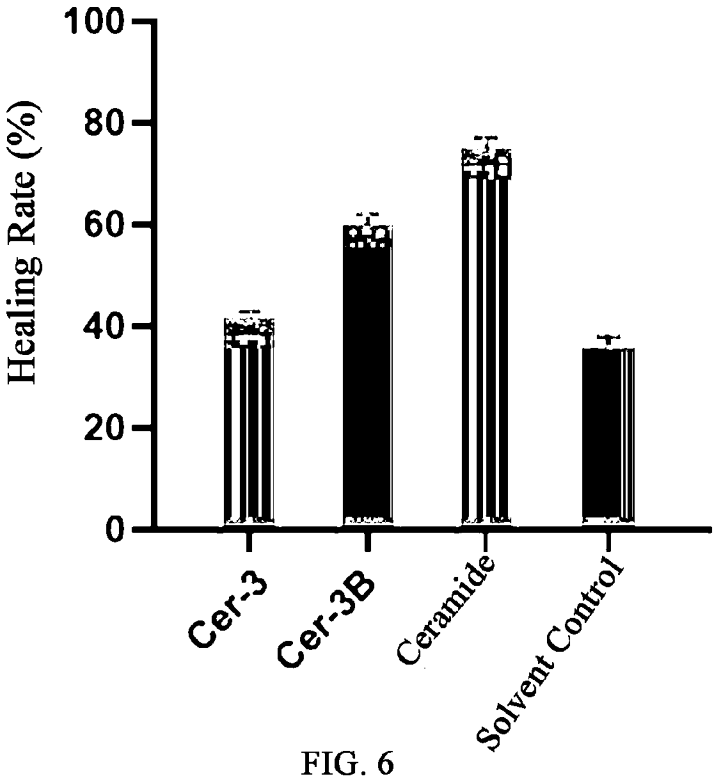
100
80
60
40
20
0
Healing Rate (%)
Cer-3
Cer-3B
Ceramide
Solvent Control

FIG. 6

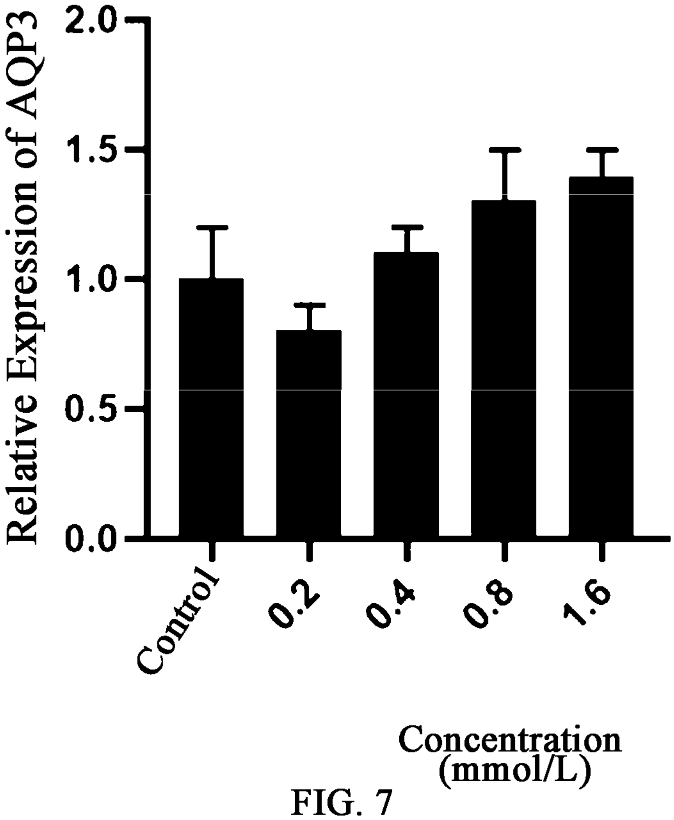
Relative Expression of AQP3
2.0
1.5
1.0
0.5
0.0
Control
0.2
0.4
0.8
1.6
Concentration
(mmol/L)

FIG. 7

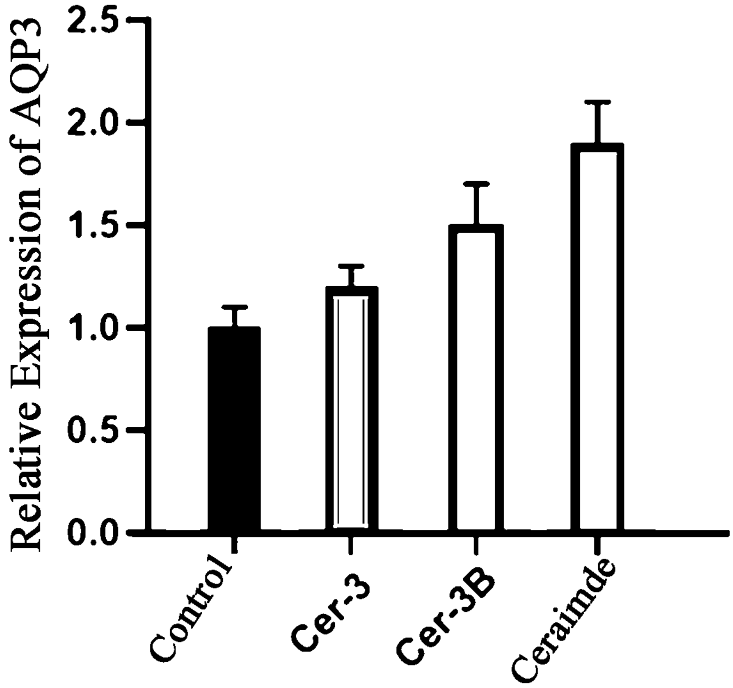
Relative Expression of AQP3
2.5
2.0
1.5
1.0
0.5
0.0
Control
Cer-3
Cer-3B
Ceraimde

FIG. 8

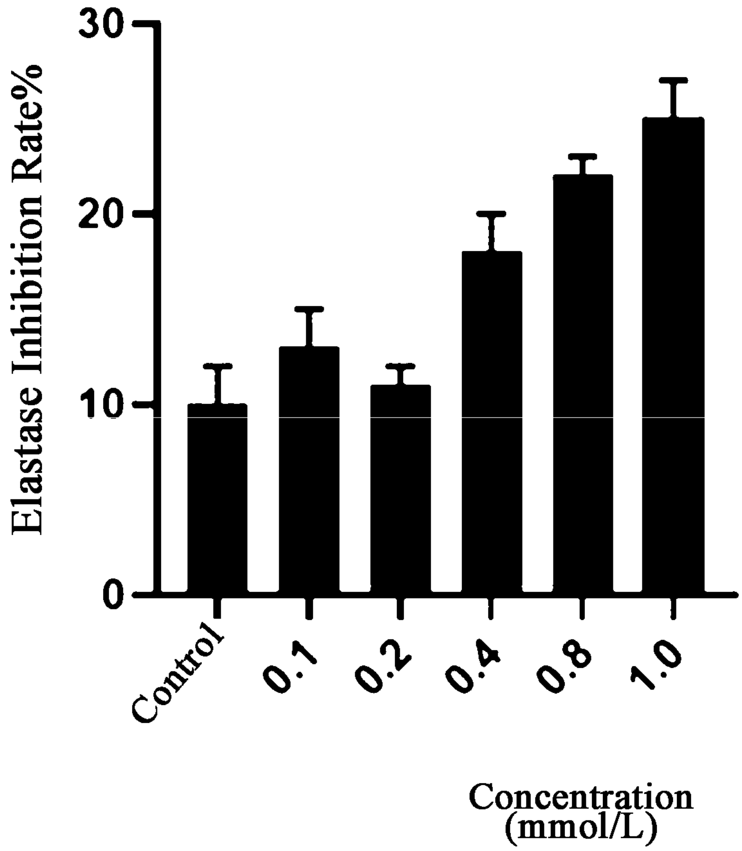
Elastase Inhibition Rate%
30
20
10
0
Control
0.1
0.2
0.4
0.8
1.0
Concentration (mmol/L)

FIG. 9

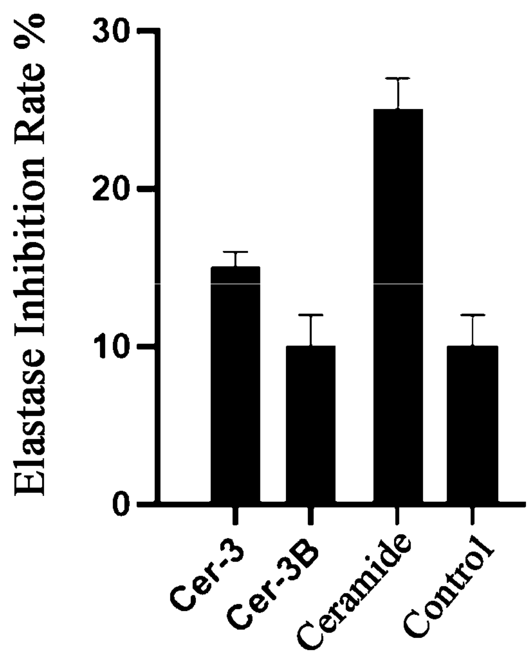
Elastase Inhibition Rate %
30
20
10
0
Cer-3
Cer-3B
Ceramide
Control

FIG. 10

**INTERNATIONAL SEARCH REPORT**

International application No. **PCT/CN2023/123721**

**A. CLASSIFICATION OF SUBJECT MATTER**

C07C231/02(2006.01)i； C07C235/08(2006.01)i； A61K8/42(2006.01)i； A61K31/231(2006.01)i； A61P29/00(2006.01)i； A61P39/00(2006.01)i； A61P39/06(2006.01)i； A61Q19/00(2006.01)i； A61Q19/08(2006.01)i； A23L33/10(2016.01)i

According to International Patent Classification (IPC) or to both national classification and IPC

**B. FIELDS SEARCHED**

Minimum documentation searched (classification system followed by classification symbols)

IPC: C07C231/-; C07C235/-; A61K8/-; A61K31/-; A61P29/-; A61P39/-; A61Q19/-; A23L33/-

Documentation searched other than minimum documentation to the extent that such documents are included in the fields searched

Electronic data base consulted during the international search (name of data base and, where practicable, search terms used)

CNABS; CNTXT; VEN; WOTXT; USTXT; EPTXT; GBTXT; CATXT; CNKI; 万方, WANFANG; STN: 神经酰胺, 分子钉, 植物鞘氨醇, 亚油酰氯, 修复, 保湿, 化妆品, 杨超文, 叶柳, 深圳市迪克曼生物科技, ceramide, phytosphingosine, TBDPSCl, linoleoyl chloride, repair+, healing, moisturizing, aging, cosmetic, 式I结构, structure of formula I, CAS号2922747-35-9, 2922747-34-8, CAS numbers 2922747-35-9 and 2922747-34-8

**C. DOCUMENTS CONSIDERED TO BE RELEVANT**

| Category* | Citation of document, with indication, where appropriate, of the relevant passages | Relevant to claim No. |
|---|---|---|
| PX | CN 115894270 A (SHENZHEN DIKEMAN BIOLOGICAL TECHNOLOGY CO., LTD.) 04 April 2023 (2023-04-04)<br>claims 1-10 | 1-10 |
| X | WO 9410131 A1 (QUEST INTERNATIONAL B.V. et al.) 11 May 1994 (1994-05-11)<br>description, page 12, compound (10), and pages 10-21 and 29, and claims 1-18 | 1-10 |
| A | KR 20170100906 A (LCS BIOTECH CO., LTD.) 05 September 2017 (2017-09-05)<br>entire document | 1-10 |
| A | CN 105037237 A (SUZHOU HIGHFINE BIOTECH CO., LTD.) 11 November 2015 (2015-11-11)<br>entire document | 1-10 |
| A | KR 20050029785 A (BIO CLUE & SOLUTION CO., LTD. et al.) 29 March 2005 (2005-03-29)<br>entire document | 1-10 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **08.11月2023(08.11.2023)** | **05 January 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/123721**

**C. DOCUMENTS CONSIDERED TO BE RELEVANT**

| Category* | Citation of document, with indication, where appropriate, of the relevant passages | Relevant to claim No. |
|---|---|---|
| A | WO 2004064823 A1 (ASTON UNIVERSITY) 05 August 2004 (2004-08-05)<br>entire document | 1-10 |
| A | CN 101434557 A (COSMOFUMU CORP.) 20 May 2009 (2009-05-20)<br>entire document | 1-10 |
| A | JP 2011195527 A (NIPPON FINE CHEMICAL CO., LTD.) 06 October 2011 (2011-10-06)<br>entire document | 1-10 |
| A | CN 110123661 A (GUANGZHOU KESIMAN BIOTECHNOLOGY CO., LTD. et al.) 16 August 2019 (2019-08-16)<br>entire document | 1-10 |
| A | US 2011213031 A1 (KANEKA CORPORATION) 01 September 2011 (2011-09-01)<br>entire document | 1-10 |

Form PCT/ISA/210 (second sheet) (July 2022)

**INTERNATIONAL SEARCH REPORT**

International application No.
**PCT/CN2023/123721**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **4-9**
because they relate to subject matter not required to be searched by this Authority, namely:

Claims 4-9 set forth the use of the ceramide of claim 1 in a health care product or a drug, which relates to the category of treatment methods for a living human or animal body, and falls within the cases set out in PCT Rule 39.1(iv) for which an international search is not required.

Nevertheless, a search is still performed on the basis of the technical subject matter of the use of the ceramide in the preparation of a health care product or a drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/123721**

| Patent document cited in search report | Publication date (day/month/year) | Patent family member(s) | Publication date (day/month/year) |
|---|---|---|---|
| CN 115894270 A | 04 April 2023 | None | |
| WO 9410131 A1 | 11 May 1994 | CA 2148139 A1 | 11 May 1994 |
| | | US 5627056 A | 06 May 1997 |
| | | AU 5373894 A | 24 May 1994 |
| | | AU 680841 B2 | 14 August 1997 |
| | | DE 69320980 D1 | 15 October 1998 |
| | | DE 69320980 T2 | 04 February 1999 |
| | | NZ 257361 A | 26 November 1996 |
| | | EP 0667853 A1 | 23 August 1995 |
| | | EP 0667853 B1 | 09 September 1998 |
| | | ES 2123066 T3 | 01 January 1999 |
| | | JPH 08502961 A | 02 April 1996 |
| KR 20170100906 A | 05 September 2017 | None | |
| CN 105037237 A | 11 November 2015 | None | |
| KR 20050029785 A | 29 March 2005 | KR 100570810 B1 | 28 April 2006 |
| WO 2004064823 A1 | 05 August 2004 | GB 0301395 D0 | 19 February 2003 |
| CN 101434557 A | 20 May 2009 | None | |
| JP 2011195527 A | 06 October 2011 | JP 5690074 B2 | 25 March 2015 |
| CN 110123661 A | 16 August 2019 | None | |
| US 2011213031 A1 | 01 September 2011 | US 8436196 B2 | 07 May 2013 |
| | | JPWO 2010038765 A1 | 01 March 2012 |
| | | JP 5513397 B2 | 04 June 2014 |
| | | WO 2010038765 A1 | 08 April 2010 |
| | | EP 2343273 A1 | 13 July 2011 |
| | | EP 2343273 A4 | 23 April 2014 |

Form PCT/ISA/210 (patent family annex) (July 2022)